# EUROPÄISCHE PATENTSCHRIFT

(11) **EP 1 467 211 B1**
(45) Veröffentlichungstag und Bekanntmachung des Hinweises auf die Patenterteilung: **30.04.2008**
(21) Anmeldenummer: 04007427.0
(22) Anmeldetag: 26.03.2004
(51) Int. Cl.: G01N 33/58, C12Q 1/42, C12Q 1/48

(54) **Verfahren zum Messen von an biologische Moleküle gebundenen, chemischen Gruppen**
Method for the measurement of chemical groups bonded to biological molecules
Procédé de mesure des groupes chimiques liés aux molécules biologiques.

(30) Priorität: 10.04.2003 CH 6532003
(43) Veröffentlichungstag der Anmeldung: 13.10.2004
(73) Patentinhaber: Tecan Trading AG, 8708 Männedorf (CH)
(72) Erfinder: Döring, Klaus, Middle Barton, Chipping Noton OX7 7BU (GB)
(74) Vertreter: OK pat AG

(56) Entgegenhaltungen:
- WO-A-00/42214
- WO-A-00/72016
- US-B1- 6 410 255

## Beschreibung

Die Erfindung betrifft ein Verfahren zum Messen der An- bzw. Abwesenheit von an biologische Moleküle gebundenen Phosphatgruppen.

Die Klassen der Enzyme, welche die Anlagerung (Phosphokinase) bzw. die Wegnahme (Phosphatasen) von Phosphatresten ah Biomolekülen katalysiert, ist von grossem biologischen und pharmakologischen Interesse. Zur Effizienzmessung der Enzymfunktion können unter anderem die Konzentration der Edukte oder Produkte der Reaktion herangezogen werden, also z.B. die Menge von phosphorylierten Biomolekülen in dem Probenvolumen.

Verfahren zum Messen der An- bzw. Abwesenheit von an biologische Moleküle gebundenen chemischen Gruppen, beispielsweise von Phosphatgruppen, sind an sich bekannt und umfassen beispielsweise die Verwendung der Fluoreszenz-Polarisations-Technik. Solche Fluoreszenz-Polarisations-Messungen beruhen auf Volumenänderungen in den gemessenen Molekülen. Viele auf einer Volumenzunahme beruhende biologische Prozesse, wie Rezeptor-Ligand-Bindungen, Antikörper-Antigen-Bindungen, DNA-Hybridisierung- oder DNA-Protein-Bindungs-Reaktionen, aber auch auf einer Volumenreduktion basierende Vorgänge, wie enzymatische Degradation, oder Dissoziations-Reaktionen können direkt mittels Fluoreszenz-Polarisation gemessen werden.

Solche Experimente entsprechen dem folgenden Schema: Ein unphosphoryliertes Biomolekül wird mit einem Enzym, das eine Phosphatgruppe an dieses Molekül anlagert, also mit einer Phosphokinase, inkubiert oder ein phosphoryliertes Peptid wird mit einem Enzym, das die Phosphatgruppe von diesem Peptid entfernt, also mit einer Phosphatase inkubiert. Die Volumenänderung eines biologischen Moleküls durch das Hinzufügen oder Wegnehmen einer kleinen chemischen Gruppe, wie z.B. einer Phosphat-, Sulfat- oder Oxalatgruppe, ist nicht genügend gross für die direkte Detektion mittels Fluoreszenz-Polarisation. Um den Volumeneffekt zu vergrössern, wird standardmässig ein Hilfsmolekül an dieses zu untersuchende biologische Molekül angehängt. Diese Hilfsmoleküle in Form von Antikörpern müssen dabei so ausgewählt sein, dass sie immer nur dann eine Bindung mit den zu untersuchenden Peptiden eingehen, wenn diese wirklich eine Phosphatgruppe aufweisen. Dadurch wird gewährleistet, dass die mittels Fluoreszenz-Polarisation zu messenden Komplexe aus Phospho-Peptiden plus Antikörpern einerseits, und nicht-phosphorylierten Peptiden ohne gebundene Hilfsmoleküle andererseits, sich in ihrem Volumen signifikant unterscheiden. Dieser kommerziell erhältliche Ansatz war ursprünglich dazu entwickelt worden, ein nicht-destruktives Signal bereit zu stellen, welches das Verfolgen der Enzymfunktion ermöglicht. In einer Situation, in der die pharmakologische Wirksamkeit von chemischen Substanzen getestet wird ("Drug Discovery Situation"), werden dem System solche Testsubstanzen zugeführt, wobei die standardmässige Messung der Fluoreszenz-Polarisation darüber Auskunft gibt, ob die Testsubstanz die gewünschte Modulationswirkung auf das Enzym zeigt oder nicht.

Alle bekannten Methoden zum Nachweis der Phosphorylierung bzw. Dephosphorylierung basieren auf dem Hinzufügen von Hilfsmolekülen, wie dies z.B. aus US 6,410,255 bekannt ist. Solche Hilfsmoleküle können neben Antikörpern auch andere chemische Verbindungen sein, welche die Phosphorylierung eines Biomoleküls erkennen und dadurch den Phosphorylierungsstatus von einem anderen unterscheiden können. Würde man als alternative Methode vorsehen, die Konzentration von anderen Edukten oder Produkten einer Phosphorylierungsreaktion zu messen, so müsste eine gegenüber der Fluoreszenz-Polarisation noch kompliziertere Additionschemie angewendet werden.

Gemäss der Aufgabe der vorliegenden Erfindung soll ein alternatives Verfahren vorgeschlagen werden, mit welchem der Nachweis für das Ablaufen einer chemischen Reaktion an einem biologischen Molekül, wie z.B. das Phosphorylieren oder Dephosphorylieren eines Peptids, auf einfache Weise erbracht werden kann.

Diese Aufgabe wird mit den Merkmalen des unabhängigen Anspruchs 1 gelöst. Bevorzugte Weiterbildungen des erfindungsgemässen Verfahrens bzw. zusätzliche erfinderische Merkmale und weitere Ausführungsbeispiele ergeben sich aus den abhängigen Ansprüchen 2 bis 10.

Es war zwar schon länger bekannt, dass sich das Signal der Fluoreszenz-Polarisation einer mit einem Fluoreszenzmarker verbundenen Probe allein durch das Hinzufügen oder Wegnehmen einer Phosphatgruppe nicht wesentlich ändert. Zudem war auch bekannt, dass der Einfluss von Ladungen die Fluoreszenz-Lebensdauer von Fluoreszenzfarbstoffen verändern kann. Dass aber das Hinzufügen einer Phosphatgruppe zu einem mit einem Fluoreszenzmarker versetzten Biomolekül genügt, um ein brauchbares Signal zu erzeugen, war absolut überraschend.

Die vorliegende Erfindung basiert somit auf der unerwarteten und überraschenden Erkenntnis, dass sich durch das Hinzufügen oder Wegnehmen einer Phosphatgruppe zu oder von einem biologischen Molekül die Fluoreszenz-Lebensdauer von an diese Proben gebundenen Fluorophoren entsprechend der An- oder Abwesenheit einer Phosphatgruppe wesentlich ändert.

Der Mechanismus, welcher der vorliegenden Erfindung zu Grunde liegt, ist nicht genau bekannt. Es wird jedoch angenommen, dass es sich um eine Interaktion zwischen dem Fluoreszenzmarker und einem geladenen Rest handelt, der nahe der Bindungsstelle des Fluoreszenzmarkers an dasselbe Biomolekül gebunden ist und/oder sich im räumlichen Schwenkbereich des Fluoreszenzmarkers befindet. Als ein Fluoreszenzmarker, dessen Fluoreszenz-Lebensdauer sich entsprechend der An- oder Abwesenheit einer Phosphatgruppe wesentlich ändert, wurde Fluoreszein gefunden. Die Erfindun umfasst die Verwendung von Fluoreszein; weiterhin wird beschrieben, dass jede chemische Reaktion eines Biomoleküls, welche einen oder mehrere geladene chemische Reste erzeugt, durch eine entsprechende Messung der Fluoreszenz-Lebensdauer nachgewiesen werden kann. Es wird davon ausgegangen, dass entsprechende oder ähnliche Resultate auch zum Nachweisen von anderen Enzym-Reaktionen verwendet werden können, falls - die Fluoreszenz-Lebensdauer von geeigneten, an die entsprechenden Biomoleküle gebundenen Fluoreszenzmarkern gemessen wird.

Bekannterweise katalysieren Enzyme die unterschiedlichsten Typen von chemischen Reaktionen. In der folgenden Tabelle werden die am häufigsten in lebenden Systemen zu beobachtenden Reaktionen aufgelistet und den Klassen von Enzymen gegenübergestellt, die bei diesen Reaktionen eine wichtige Rolle spielen:

| **Reaktionstyp** | **betrifft** | **Enzymklasse** | **Enzymbezeichnung** |
|---|---|---|---|
| Oxidation; Reduktion | C-O, C-C oder C-N-Bindungen | I | Oxidoreduktasen |
| Intramolekular-; Intermoiekuiar-Transfer | Transfer von funktionellen Gruppen | II | Transferasen |
| Hydrolyse | Ester, Ether und Amide | III | Hydrolasen |
| Elimination; Addition | C-O , C-C oder C-N-Bindungen | IV | Lyasen |
| Isomersation | Sterische Veränderungen | V | Isomerasen |
| Bindungen | Ester-, Thiolester- und Amid-Bindungen | VI | Ligasen |

Unter Verwendung des erfindungsgemässen Verfahrens können Enzymreaktionen nachgewiesen und quantifiziert werden, die durch einen katalytischen Prozess die Anreicherung von Phosphatgruppen bewirken.

### Enzymklasse I:

Oxidoreduktasen katalysieren Redox-Reaktionen, wobei eine Oxidation den Verlust von Elektronen und eine Reduktion den Gewinn von Elektronen bedeutet. So ist es zum Beispiel möglich, dass bei einem Fluoreszenzmarker, der sich in der unmittelbaren Nachbarschaft zum aktiven Zentrum einer Oxidoreduktase befindet, eine Änderung im Fluoreszenzlebensdauersignal beobachtet werden kann, welche auf der Wanderung der Elektronen durch die unterschiedlichen Seitenketten der Aminosäuren innerhalb des Enzyms oder des Substrats während der katalytischen Umwandlung beruht. Diese Fluoreszenzkette wurde bereits in Enzymen der Klasse I nachgewiesen: Bei der Reduktion von NAD⁺ zu NADH mittels Dehydrogenasen ergibt sich, dass NAD⁺ im Gegensatz zu NADH nicht-fluoreszierend ist.

### Enzymklasse II:

Transferasen sind Enzyme, welche den Transfer einer funktionellen Gruppe von einem Substituenten zu einem anderen katalysieren, was innerhalb des selben Moleküls oder auch zwischen verschiedenen Molekülen passieren kann. Typische Vertreter dieser Klasse sind Kinasen, welche ein ATP zu einem Protein oder Peptid transferieren. Ähnliche Effekte bezüglich der Fluoreszenzlebensdauer wie bei den weiter unten beschriebenen Phosphokinase-Reaktionen werden auch bei Thiolasen und anderen Transferasen erwartet.

### Enzymklasse III:

Hydrolasen sind Enzyme, welche die Hydrolyse von Karbonsäureestern, Hemiacetalethern (Zuckerverbindungen), Thioethern, Amiden (Peptidbindungen) und Säureanhydriden katalysieren. In diese Gruppe gehören auch die Phosphatasen, weil diese eine Phosphatgruppe ins Wasser abspalten. Die Ausführungen zur Klasse I und II treffen auch hier zu.

### Enzymklasse IV:

Lyasen sind Enzyme, welche die Entfernung (Elimination) oder die Hinzufügung (Addition) von chemischen Gruppen katalysieren. Als ein Beispiel sei hier die Entfernung von einer CO-Gruppe, also die Decarboxylierung genannt. Weil die Decarboxylasen typischerweise ein Elektronenpaar abgeben um die Decarboxylierungsreaktion zu starten, weil also eine Veränderung der "elektronischen Signatur" des Substrats im Laufe der Decarboxylierungsreaktion erwartet werden kann, sollte auch diese Klasse von Reaktionen bzw. Enzymen mittels der Lebensdauerfluorometrie untersucht werden können.

### Enzymklasse V:

Isomerasen katalysieren gewisse intramolekularen Umgruppierungen, wie Racemisierung, Epimerisation, Cis-Trans-Conversionen und Enol-Keto-Tautomerisationen. Weil auch bei Enol-Keto-Tautomerisationen massive Elektronenbewegungen zu erwarten sind, sollte auch hier eine Veränderung der Fluoreszenzlebensdauer gemessen werden können.

### Enzymklasse VI:

Ligasen sind Enzyme, welche eine Verbindung zwischen Molekülen katalysieren, wobei sie die Energie verwenden, welche sie aus der Spaltung von ATP (oder einem ähnlichen Nukleosidtriphosphat) gewinnen. In diese Klasse gehören beispielsweise Fettsäuresynthetasen und DNA-Polymerasen. Auch bei diesen Enzymen ist mit grosser Wahrscheinlichkeit anzunehmen, dass eine durch diese Enzyme katalysierte Reaktion eine signifikante Änderung der Fluoreszenzlebensdauer eines Fluorophors bewirkt, der sich nahe des aktiven Zentrums des Enzyms befindet.

Gemäss der vorliegenden Erfindung genügt ein einziger Fluoreszenzmarker, der sich in der Nähe und/oder im Einflussbereich einer chemisch aktiven Gruppe am Biomolekül befindet. Bedingung für den Fluoreszenzmarker ist, dass er auf eine Änderung der Ladungsdichte und/oder Konfiguration in seiner unmittelbaren molekularen Umgebung mit einer Verschiebung der Fluoreszenz-Lebensdauer reagiert. Das dadurch generierte Signal ist deshalb viel direkter als das auf dem Hinzufügen und Anbinden von Hilfsmolekülen angewiesene Verfahren aus dem Stand der Technik.

Als biologische Moleküle (mit natürlichem oder künstlichem Ursprung) werden im Zusammenhang mit der vorliegenden Erfindung hier lediglich beispielsweise und keinesfalls abschliessend aufgezählt:
- eine Aminosäurensequenz umfassende biologische oder künstlich erzeugte Moleküle, wie Proteine, Peptide, Glykoproteine und Lipoproteine;
- eine Nukleinsäurensequenz umfassende biologische oder künstlich erzeugte Moleküle wie, DNA- und RNA-Stücke oder Oligonukleotide;
- andere auf einem biologischen Prozess beruhende oder für einen solchen dienende Moleküle wie zyklisches Adenosinmonophosphat (AMP) oder Guanosinmonophoshat (GMP);
- Monozucker, Mehrfachzucker und andere Makromoleküle.

Unter einer Multiwellplatte wird im Zusammenhang mit der vorliegenden Erfindung eine Anordnung von offenen oder geschlossenen Kammern verstanden. Diese Anordnung ist vorzugsweise regelmässig und stellt ein gitterförmiges Array von Probenbehältern oder Probenträgern dar. Bekannte solche Multiwellplatten sind z.B. die sogenannten Mikroplatten mit 96, 384 oder 1536 in einem rechtwinkligen Gitter angeordneten Wells. Es müssen aber nicht unbedingt Vertiefungen sein, in denen die Proben angeordnet sind. Kleinere Proben können auch auf einer Ebene in einem Array angeordnet sein und unter sich lediglich durch hydrophobe Bereiche oder kleine Erhebungen abgetrennt sein. Vorzugsweise ist allen Multiwellplatten gemeinsam, dass eine grosse Zahl von Proben mit einem adressierbaren Standort im wesentlichen gleichzeitig oder sogar simultan verarbeitet werden können

Zum Messen der Fluoreszenz-Lebensdauer sind unter anderem die Phasenmodulationstechnik und das "Time Correlated Single Photon Counting (TCSPC)" bekannt. An Hand eines ausgewählten und beispielhaften Experiments soll nun die vorliegende Erfindung näher erläutert werden.

### Experiment 1

Bei diesem Experiment wurde die an sich ebenfalls bekannte Methode des zeitabhängigen Einzelphotonen-Zählens, d.h. des "Time Correlated Single Photon Counting (TCSPC)" zum Messen der Fluoreszenz-Lebensdauer angewendet.

### A) Technische Details:

Die Experimente wurden mit kommerziell erhältlichen Chemikalien ausgeführt. Dabei wurden folgende Chemikalien verwendet:
PTK-Grün als Tracerpeptid, Konzentration 2 nM, P-2843 von PANVERA (PANVERA, 501 Charmany Drive, Madison WI USA), als Teil des Kinase-Assays "Grün", gemäss Kit Nr. P-2837;
PTP1B Phosphatase, SE-332 von BIOMOL (BIOMOL Research Laboratories, Inc., 5120 Butler Pike, Plymouth Meeting PA USA 19462-1202);
Fluoreszein und PBS-Puffer.

Die Proben wurden in schwarzen 384-Well Mikroplatten von GREINER (GREINER Bio-One GmbH, Bad Haller Strasse 32, 4550 Kremsmünster, Österreich) mit einem Füllvolumen von 70 µl präpariert. Die Enzymkonzentration betrug 100 pM bis 100 nM. Inkubiert wurde während einer Dauer von 30 Min.

Zum Messen der Fluoreszenz-Lebensdauer wurde ein Gerät "Ultra Evolution" mit Fluoreszenz-Lebensdauer ("fluorescence life time" = FLT) - Option von TECAN (TECAN Austria GmbH, Grödig, Salzburg, Österreich) verwendet. Der Laser für die Anregung der Fluoreszenz arbeitete bei 440 nm Wellenlänge und einer Wiederholfrequenz von 20 MHz. Der Emissionsfilter war für eine Wellenlänge von 544 nm und eine Bandbreite von 25 nm ausgelegt und die Integrationszeit pro Well betrug 1 Sekunde.

### B) Resultate:

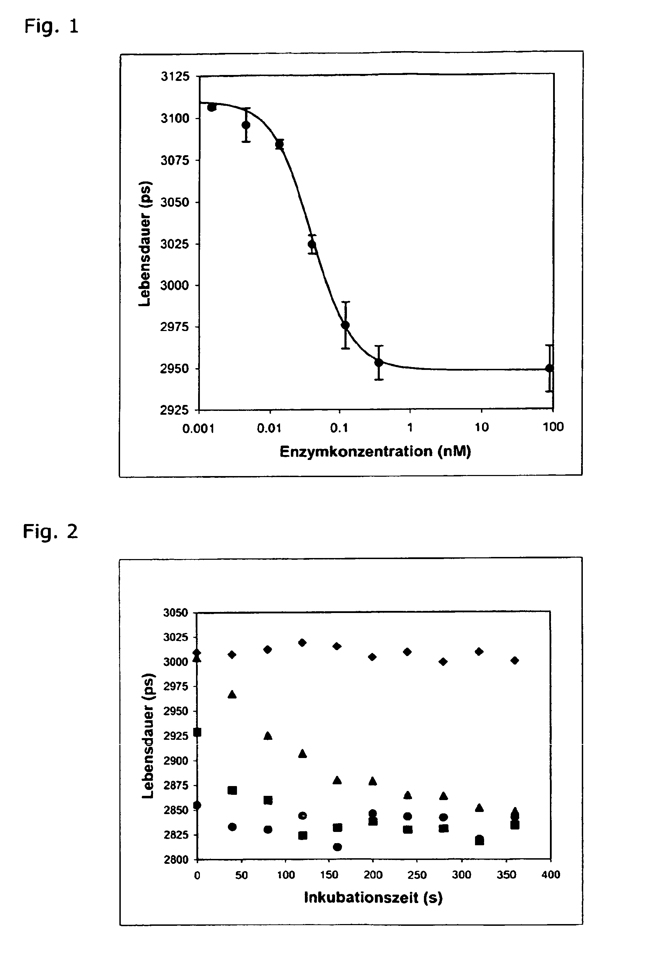

Figur 1 zeigt die gemessene Fluoreszenz-Lebensdauer des Fluoreszenzmarkers Fluoreszein, der ein Teil des Tracer-Peptids (Biomolekül) ist, in Abhängigkeit der Enzymkonzentration. Es wird hier ausdrücklich darauf hingewiesen, dass kein zusätzliches Hilfsmolekül (wie ein Antikörper und dergleichen) verwendet werden musste, um diese Messresultate zu erhalten. Die Messung wurde 30 Minuten nach dem Hinzufügen des Enzyms PTP1B zum Tracer-Peptid ausgeführt. Die Fluoreszenz-Lebensdauer entspricht dem Status der Phosphorylierung des Peptides, wobei höhere Enzym-Konzentrationen schneller arbeiten und mehr dephosphoryliertes Peptid produzieren. Das an die nun dephosphorylierten Peptide gebundene Fluoreszein zeigt dagegen eine kürzere Lebensdauer.

Nach sehr langer Zeit würden - unabhängig von der Enzymkonzentration - alle Phosphatgruppen vom Tracer-Peptid entfernt sein. Allerdings bestimmt die Enzymkonzentration die genaue Länge dieser Zeit.

Figur 1 zeigt zudem, dass bei sehr tiefer Enzymkonzentration der Phosphorylierungszustand nicht sehr verändert wird; wo hingegen bei den höchsten verwendeten Konzentrationen durch Zugabe von noch mehr Enzymen kein zusätzlicher Effekt mehr erzeugt werden kann. In diesem Experiment wurde das ganze Substrat der Probe während der Inkubationszeit verarbeitet, so dass im Mittelbereich das Verhältnis der apparenten Enzymfunktion sichtbar wird. Figur 1 basiert auf Daten zur Dephosphorylierung des Panvera Tracers P2843 bei unterschiedlichen Phosphatasekonzentrationen. Die Fehlerbalken ergeben sich aus drei unabhängigen Messungen. Die durchgezogenen Linie ist nur eine Hilfe zur leichteren Sichtbarmachung der Abhängigkeit. Die Lage dieser Linie verschiebt sich mit kürzerer Inkubationszeit nach rechts, bei längerer Zeit nach links. Der z'-Wert für den in Figur 1 gezeigten Datensatz beträgt 0.62.

Figur 2 zeigt die Zeitabhängigkeit der Enzymreaktion, d.h. die Enzymkinetk für verschiedene Enzymkonzentrationen. Ohne Enzym bleibt das Lebensdauersignal stabil, d.h. es findet keine Dephosphorylierung statt. Bei mittlerer Konzentration sieht man in dem gewählten Zeitabschnitt von 6 Minuten den gesamten Verlauf, für sehr hohe Konzentrationen verläuft die Reaktion zu schnell, um in diesem Experiment noch messbar zu sein. Die den unterschiedlichen Enzymkonzentrationen entsprechenden Messpunkte sind als Rauten (ohne Enzym), Dreiecke (1 nM), Quadrate (3 nM) oder Punkte (30 nM) dargestellt.

Zur Kontrolle dieser Resultate wurden zwei Proben mit unterschiedlichem Fluoreszenz-Lebensdauer-Signal mittels Fluoreszenz-Polarisation gemessen; es konnte mit dieser bekannten Methode kein Unterschied in der Phosphorylierung der Tracer-Peptide festgestellt werden.

Experimente wie das eben beschriebene spielen eine wichtige Rolle in der Versuchs-Entwicklung ("Assay-Development") oder in einem Hochdurchsatz-Testlabor ("High Throughput Screening Laboratory"). Allerdings ermöglichten die bisher bestehenden Apparaturen in solchen Labors das Messen der Fluoreszenz-Lebensdauer nicht.

Wie eingangs dargelegt, ist eine Enzymreaktion, bei der ein Peptid phosphoryliert oder dephosphoryliert wird, von grossem biologischen und pharmakologischen Interesse. Die vorliegende Erfindung ermöglicht einen neuen Weg, derart wichtige Vorgänge zu untersuchen und darzustellen. Speziell bevorzugte Verwendungen dieses erfindungsgemässen Verfahrens umfassen das Screening im Zusammenhang mit dem "Drug Discovery", also der Entdeckung, Erforschung, Optimierung oder Validierung bzw. dem Nachweis von pharmakologisch wirksamen Substanzen und/oder im Zusammenhang mit der entsprechenden Arzneimittelherstellung.

Im Zusammenhang mit der hier vorliegenden Erfindung muss das Anbringen von Phosphatgruppen an ein Biomolekül direkten Einfluss auf den Fluoreszenzfarbstoff nehmen. Dazu müssen beide Gruppen entweder in räumlicher Nähe zueinander sein, oder der Einfluss kann durch eine Konformationsänderung des Biomoleküls vermittelt werden, die dann ihrerseits die molekulare Umgebung des Farbstoffs verändert. Beide chemischen Einheiten, die Phosphatgruppe sowie der Farbstoff, müssen am selben Biomolekül , z.B. kovalent, gebunden sein. Allerdings darf das Biomolekül auch aus mehreren Untereinheiten bestehen, also (Hetero- oder Homo-) Dimere, Trimere, allgemein Oligomere. In diesem Fall können die beiden Reste auch auf verschiedenen Untereinheiten des selben Biomoleküls sitzen. Weitere Ausführungsbeispiele sind in den abhängigen Ansprüchen 2 bis 10 dargestellt.

## Patentansprüche

1. Verfahren zum Messen des Status der Phosphorylierung von biologischen Molekülen in einer Probe, bei welchem diese Moleküle mit Fluoreszenzmarkern markiert werden und diese Fluoreszenzmarker mittels Bestrahlung der Probe mit Licht aktiviert werden, **dadurch gekennzeichnet, dass** das Verfahren folgende Schritte umfasst:
(a) Auswählen eines Fluoreszenzmarkers zum Anbinden an ein Biomolekül, dessen Fluoreszenz-Lebensdauer je nach An- oder Abwesenheit von an diesem Biomolekül gebundenen Phosphatgruppen einen unterschiedlichen Wert annimmt;
(b) Binden des in Schritt (a) ausgewählten Fluoreszenzmarkers an ein Biomolekül;
(c) Messen der Fluoreszenz-Lebensdauer des gemäss Schritt (b) an ein Biomolekül gebundenen Fluoreszenzmarkers;
(d) Klassifizierung der Biomoleküle in der Probe gemäss der An- bzw. Abwesenheit von an diese Biomoleküle gebundenen Phosphatgruppen,
wobei diese Klassifizierung auf der jeweils unterschiedlichen Fluoreszenz-Lebensdauer des Fluoreszenzmarkers basiert.

2. Verfahren gemäss Anspruch 1, **dadurch gekennzeichnet, dass** die biologischen Moleküle ausgewählt werden aus einer Gruppe, die eine Aminosäurensequenz umfasst, wie Proteine, Peptide, Glykoproteine und Lipoproteine.

3. Verfahren gemäss einem der vorhergehenden Ansprüche, **dadurch gekennzeichnet, dass** der Fluoreszenzmarker Fluoreszein ist.

4. Verfahren gemäss einem der vorhergehenden Ansprüche, **dadurch gekennzeichnet, dass** die biologischen Moleküle einer Probe vor einer Messung des Phosphorylierungszustandes mit einer Phosphatase oder mit einer Phosphokinase inkubiert werden.

5. Verfahren gemäss einem der vorhergehenden Ansprüche, **dadurch gekennzeichnet, dass** das Markieren von biologischen Molekülen und/oder das Aktivieren der Probe und/oder das Messen der Fluoreszenz-Lebensdauer in einer Multiwellplatte, wie einer Mikroplatte mit 96, 384 oder 1536 Wells, durchgeführt und ein Rechner zum automatischen Klassifizieren der Biomoleküle bzw. der Proben verwendet wird.

6. Verfahren nach einem oder mehreren der vorhergehenden Ansprüche, **dadurch gekennzeichnet, dass** das Messen der Fluoreszenz-Lebensdauer mittels Time Correlated Single Photon Counting (TCSPC) oder mittels der Phasenmodulationstechnik durchgeführt wird.

7. Verfahren nach einem oder mehreren der vorhergehenden Ansprüche, **dadurch gekennzeichnet, dass** der Anteil der beiden Spezies der Biomoleküle in der Probe an Hand einer Kalibrierung quantifiziert wird.

8. Verwendung des Verfahrens gemäss einem oder mehreren der Ansprüche 1 bis 7 zum Screening von chemischen Wirkstoffen für die Entdeckung (Drug Discovery) von pharmakologisch wirksamen Substanzen und/oder für die Arzneimittelherstellung.

9. Verwendung des Verfahrens gemäss einem oder mehreren der Ansprüche 1 bis 7 zum Nachweis von Defekten in menschlichen oder tierischen Enzymen.

10. Verwendung des Verfahrens gemäss einem oder mehreren der Ansprüche 1 bis 7 zum Nachweisen und/oder Quantifizieren einer Phosphorylierungs- oder Dephosphorylierungs-Reaktion von Enzymen.

## Claims

1. A method for measuring the status of phosphorylation of biological molecules in a sample, in which these molecules are marked using fluorescence markers and these fluorescence markers are activated by irradiation of the sample with light, **characterized in that** the method comprises the following steps:
(a) selecting a fluorescence marker for binding to a biomolecule, whose fluorescence lifetime assumes different values depending on the presence or absence of phosphate groups bound to this biomolecule;
(b) binding the fluorescence marker selected in step (a) to a biomolecule;
(c) measuring the fluorescence lifetime of the fluorescence marker bound to a biomolecule according to step (b);
(d) classifying the biomolecules in the sample according to the presence or absence of phosphate groups bound to these biomolecules, this classification being based on the particular differing fluorescence lifetime of the fluorescence marker.

2. The method according to claim 1, **characterized in that** the biological molecules are selected from a group which comprises an amino acid sequence, such as proteins, peptides, glycoproteins, and lipoproteins.

3. The method according to one of the preceding claims, **characterized in that** the fluorescence marker is fluorescein.

4. The method according to one of the preceding claims, **characterized in that** the biological molecules of a sample are incubated with a phosphatase or with a phosphokinase prior to a measurement of the phosphorylation status.

5. The method according to one of the preceding claims, **characterized in that** the marking of biological molecules and/or the activation of the sample and/or the measurement of the fluorescence lifetime is performed in a multiwell plate, such as a microplate having 96, 384, or 1536 wells, and a computer is used for automatically classifying the biomolecules or the samples.

6. The method according to one or more of the preceding claims, **characterized in that** the measurement of the fluorescence lifetime is performed using Time Correlated Single Photon Counting (TCSPC) or using the phase modulation technique.

7. The method according to one or more of the preceding claims, **characterized in that** the proportion of the two species of the biomolecules in the sample is quantified on the basis of a calibration.

8. A use of the method according to one or more of the claims 1 through 7 for screening chemical active ingredients for the discovery of pharmacologically active substances (drug discovery) and/or for the production of pharmaceuticals.

9. A use of the method according to one or more of the claims 1 through 7 for detecting defects in human or animal enzymes.

10. A use of the method according to one or more of the claims 1 through 7 for detecting and/or quantifying a phosphorylation or dephosphorylation reaction of enzymes.

## Revendications

1. Procédé de mesure de l'état de la phosphorylation de molécules biologiques dans un échantillon, dans lequel ces molécules sont marquées avec des marqueurs de fluorescence et ces marqueurs de fluorescence sont activés au moyen d'une irradiation de l'échantillon avec de la lumière, **caractérisé en ce que** le procédé comprend les étapes suivantes :
(a) sélection d'un marqueur de fluorescence permettant de se lier à une biomolécule, dont la durée de vie de fluorescence prend une valeur différente en fonction de l'absence ou de la présence de groupes phosphate liés à cette biomolécule ;
(b) liaison du marqueur de fluorescence sélectionné dans l'étape (a) à une biomolécule ;
(c) mesure de la durée de vie de la fluorescence du marqueur de fluorescence lié à la biomolécule selon l'étape (b) ;
(d) classification des biomolécules de l'échantillon selon la présence ou l'absence de groupes phosphate liés à ces biomolécules, **en ce que** cette classification est fondée sur la différente durée de vie de la fluorescence respective du marqueur de fluorescence.

2. Procédé selon la revendication 1, **caractérisé en ce que** les molécules biologiques sont sélectionnées dans un groupe qui comprend une séquence d'acides aminés comme des protéines, des peptides, des glycoprotéines et des lipoprotéines.

3. Procédé selon l'une des revendications précédentes, **caractérisé en ce que** le marqueur de fluorescence est la fluorescéine.

4. Procédé selon l'une des revendications précédentes, **caractérisé en ce que** les molécules biologiques d'un échantillon sont mises à incuber, avant une mesure de l'état de phosphorylation, avec une phosphatase ou avec une phosphokinase.

5. Procédé selon l'une des revendications précédentes, **caractérisé en ce que** le marquage de molécules biologiques et/ou l'activation de l'échantillon et/ou la mesure de la durée de vie de la fluorescence est conduit dans une plaque multi-puits, comme une plaque de microtitration 96, 384 ou 1 536 puits, et **en ce qu'**on utilise un ordinateur pour classer automatiquement les biomolécules ou les échantillons.

6. Procédé selon une ou plusieurs des revendications précédentes, **caractérisé en ce que** la mesure de la durée de vie de fluorescence est conduit au moyen d'un comptage de photon unique corrélé au temps (ou TCSPC, Time Correlated Single Photon Counting) ou au moyen d'une technique de modulation de phase.

7. Procédé selon une ou plusieurs des revendications précédentes, **caractérisé en ce que** la fraction des deux espèces de biomolécules dans l'échantillon est quantifiée au moyen d'un étalonnage.

8. Utilisation du procédé selon une ou plusieurs des revendications 1 à 7 afin de cribler des substances actives chimiques pour la découverte (drug discovery) de substances efficaces sur le plan pharmacologique et/ou pour la fabrication de médicaments.

9. Utilisation du procédé selon une ou plusieurs des revendications 1 à 7 pour détecter des défauts d'enzymes humaines ou animales.

10. Utilisation du procédé selon une ou plusieurs des revendications 1 à 7 pour détecter et/ou quantifier une réaction de phosphorylation ou de déphosphorylation d'enzymes.
